# EUROPEAN PATENT APPLICATION

(11) **EP 1 544 302 A1**
(43) Date of publication of application: **22.06.2005**
(21) Application number: 03784601.1
(22) Date of filing: 08.08.2003
(51) Int. Cl.: C12N 15/53, A01K 67/027

(54) **KNOCKIN NONHUMAN ANIMAL AND TISSUE-SPECIFIC MnSOD-KNOCKOUT NONHUMAN ANIMAL**

(30) Priority: 09.08.2002 JP 2002232625
(71) Applicant: Life Science Solutions Co., Ltd., Tokyo 150-0012 (JP)
(72) Inventor: SHIRASAWA, Takuji, Tokyo 177-0043 (JP); KOSEKI, Haruhiko, Chiba-shi, Chiba 260-0856 (JP)
(74) Representative: Minderop, Ralph H., Dr. rer. nat.
(86) International application number: PCT/JP2003/010118
(87) International publication number: WO 2004/014131

(57) **Abstract**

A knockin nonhuman animal having a DNA wherein a region containing at least an exon of an MnSOD gene is interposed between two recombinase recognition sequences is disclosed. Further, a knockout nonhuman animal obtainable by mating a transgenic nonhuman animal expressing a recombinase in a specific tissue with the above knockin nonhuman animal, wherein an expression of manganese superoxide dismutase in the specific tissue is suppressed or deficient; and a nonhuman animal obtainable by further mating one of the above animals with the above knockout nonhuman animal, wherein manganese superoxide dismutase is specifically deficient in the specific tissue are disclosed.

SEQUENCE LISTING

<110>  SHIRASAWA, Takuji
<110>  KOSEKI, Akihiko

<120>  Knockin non-human animal and tissue-specific MnSOD knockout non-h
uman animal

<130>  TMI-694

<150>  JP 2002-232625
<151>  2002-08-09

<160>  9

<170>  PatentIn version 3.1

<210>  1
<211>  33
<212>  DNA
<213>  artificial sequence

<220>
<223>  Description of Artificial Sequence: an artificially synthesized
       primer sequence

<400>  1
ataagaatgc ggccgctgac ggaccttact gtt                                33


<210>  2
<211>  28
<212>  DNA
<213>  artificial sequence

<220>
<223>  Description of Artificial Sequence: an artificially synthesized
       primer sequence

<400>  2
acgcgtcgac agatgccaga tgtcacct                                      28


<210>  3

<211>  28
<212>  DNA
<213>  artificial sequence

<220>
<223>  Description of Artificial Sequence: an artificially synthesized
       primer sequence

<400>  3
ccgctccagc tgagccatct cactagcc                                          28


<210>  4
<211>  26
<212>  DNA
<213>  artificial sequence

<220>
<223>  Description of Artificial Sequence: an artificially synthesized
       primer sequence

<400>  4
acgcgtcgac agctacattg cactca                                            26


<210>  5
<211>  27
<212>  DNA
<213>  artificial sequence

<220>
<223>  Description of Artificial Sequence: an artificially synthesized
       primer sequence

<400>  5
acgcgtcgac tgttgtccag acatctt                                           27


<210>  6
<211>  28
<212>  DNA
<213>  artificial sequence

<220>

<223>  Description of Artificial Sequence: an artificially synthesized
      primer sequence

<400>  6
acgcgtcgac cagcaacaca tggtggct                                          28


<210>  7
<211>  22
<212>  DNA
<213>  Mus sp.

<400>  7
cgaggggcat ctagtggaga ag                                                22


<210>  8
<211>  23
<212>  DNA
<213>  Mus sp.

<400>  8
ttagggctca ggtttgtcca gaa                                               23


<210>  9
<211>  17
<212>  DNA
<213>  Escherichia coli

<400>  9
agcttggctg gacgtaa                                                      17

## Description

### TECHNICAL FIELD

The present invention relates to a knockin nonhuman animal (for example, a knockin mouse) having a DNA in which an exon of a manganese superoxide dismutase (hereinafter referred to as MnSOD) gene is interposed between recombinase recognition sequences (for example, a loxp sequence), and a tissue-specific knockout nonhuman animal obtainable by mating the knockin nonhuman animal with a transgenic nonhuman animal (for example a transgenic mouse) expressing a recombinase (for example, Cre recombinase) in a specific tissue (particularly, a mouse in which MnSOD is specifically deficient in the specific tissue).

### BACKGROUND ART

Senescence in animals progresses with aging, and reactive oxygen theory is proposed as the mechanism of aging. It was reported that reactive oxygen species are involved in various diseases caused by the accumulation of reactive oxygen species, such as an ischemia reperfusion injury of the brain or heart, Parkinson disease, neurodegenerative disease, or arteriosclerosis.

Reactive oxygen species are byproducts of the internal respiration in mitochondria. MnSOD converts the reactive oxygen species in mitochondria, superoxide, to hydrogen peroxide. To investigate the role of MnSOD in the living body, the effects of reactive oxygen species, or causes of the above diseases, tissue-specific MnSOD-gene-deficient model mice showing an accumulation of reactive oxygen species were generated in an attempt to clarify the mechanism of diseases caused by the accumulation of reactive oxygen species.

For example, it was found that mice deficient in exon 3 of MnSOD showed dilated cardiomyopathy, neurodegeneration, or a reduction in the activity of mitochondrial respiratory enzymes, and the mice died in the neonatal period [Nat. Genet., 11, 376-381 (1995)]. Further, it was found that MnSOD knockout mice showed severe anemia or a degeneration of neurons in the basal ganglia, and that mice older than 7 days exhibit a mitochondrial injury, and died within 10 days [Proc. Natl. Acad. Sci. USA, 93, 9782-9787 (1996)].

### DISCLOSURE OF THE INVENTION

A technique for generating transgenic mice, the gene targeting method, is widely used to generate model mice [Nature, 1988 Nov 24; 336 (6197)]. In the method, a targeting vector is constructed, and a homologous recombination is carried out in embryonic stem (ES) cells to generate transgenic mice.

A recombinase derived from bacteriophage P1, Cre protein, recognizes loxp sequences (34 base pairs) and excises a sequence interposed between the loxp sequences. In the present invention, the mechanism is used to generate, for example, a flox animal in which the loxp sequences are inserted into the upstream and downstream regions of exon 3 of the MnSOD gene, by the gene targeting method. The flox mouse may be mated with a transgenic mouse overexpressing Cre protein tissue-specifically, to generate a conditional knockout mouse deficient in the MnSOD gene tissue-specifically.

Hitherto, it was difficult to model the effects of reactive oxygen species on each organ separately. Model mice systemically deficient in MnSOD were reported, but it was impossible to evaluate the effects of reactive oxygen species over a long time because the mice died in the neonatal period. Further, it is difficult to evaluate the sensitivity of each organ to reactive oxygen species or an amount of reactive oxygen species generated in each organ. In a certain organ, even if the sensitivity to reactive oxygen species is low, it is impossible to deny the possibility that degeneration in the conventional model mice is caused by systemic changes. As described above, it is difficult to evaluate aging phenomena in each organ using the conventional model mice, and impossible to analyze diseases in which morbidity is increased with senescence and specific changes occur in an organ (for example, Parkinson disease or arteriosclerosis).

Therefore, an object of the present invention is to provide a long-lived knockout nonhuman animal (such as knockout mouse) in which the MnSOD gene is tissue-specifically suppressed or deficient (particularly deficient), and a knockin nonhuman animal (preferably knockin mouse) which can be used to generate the knockout nonhuman animal. The knockout mouse is a model mouse capable of evaluating the effects of reactive oxygen species by the deficiency of the MnSOD gene in adult tissues or the normal aging process.

In the present invention, the inventors used the Cre-loxp system to provide tissue-specific MnSOD-deficient mouse, and knockin mouse capable of generating the tissue-specific MnSOD-deficient mice. The knockin mouse usually exhibits the same phenotype as that of the wild-type, with respect to the expression of MnSOD, but the MnSOD gene is deleted (i.e., knockout) when Cre recombinase is expressed in the model mouse. Cre recombinase can be expressed by mating the model mouse with a transgenic mouse overexpressing Cre recombinase. Further, the model mouse is mated with a tissue-specific transgenic mouse overexpressing Cre recombinase to generate a conditional knockout mouse in which the MnSOD gene is tissue-specifically deleted. By using the knockout mouse, it is possible to analyze adult tissues which cannot be analyzed by the conventional systemic knockout mouse.

Accordingly, the present invention relates to a knockin nonhuman animal having a DNA wherein a region containing at least an exon of a manganese superoxide dismutase gene is interposed between two recombinase recognition sequences.

According to a preferred embodiment of the present invention, the exon is exon 3. According to another preferred embodiment of the present invention, a marker gene (more preferably, a neomycin resistance gene) is interposed between the two recombinase recognition sequences. According to still another preferred embodiment of the present invention, the recombinase recognition sequence is a loxp sequence. According to still another preferred embodiment of the present invention, the nonhuman animal is a mouse.

The present invention relates to a knockout nonhuman animal obtainable by mating the above knockin nonhuman animal with a transgenic nonhuman animal expressing a recombinase in a specific tissue, wherein an expression of manganese superoxide dismutase in the specific tissue is suppressed or deficient.

The present invention relates to a knockout nonhuman animal obtainable by mating the above knockout nonhuman animal with the above knockin nonhuman animal or the above knockout nonhuman animal, wherein manganese superoxide dismutase is specifically deficient in the specific tissue.

According to a preferred embodiment of the present invention, the recombinase is Cre recombinase.

The present invention relates to a DNA wherein a region containing at least an exon of a manganese superoxide dismutase gene is interposed between two recombinase recognition sequences.

### BRIEF DESCRIPTION OF THE DRAWINGS

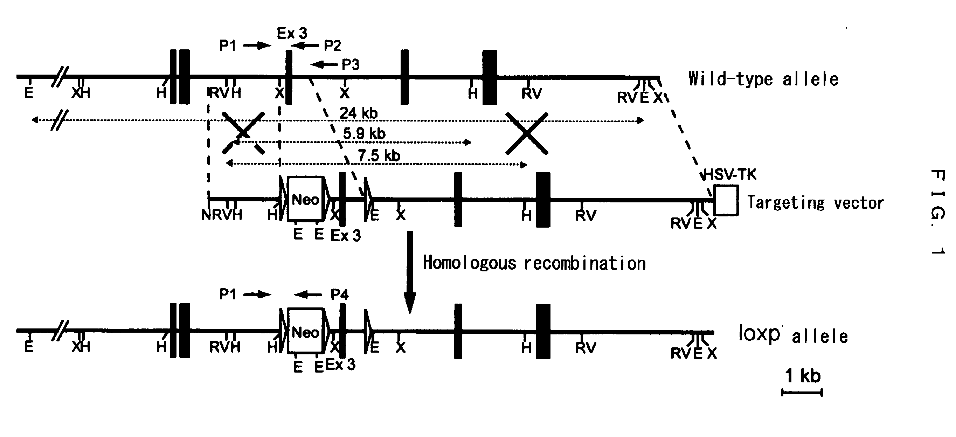

Figure 1 schematically shows the strategy for generation of knockin mice in which exon 3 of the MnSOD gene is interposed between two loxp sequences. The top line represents the MnSOD gene containing five exons, the middle line represents a targeting construct, and the bottom line represents an allele after homologous recombination. The solid boxes represent exons, and "Ex3" represents exon 3 of the MnSOD gene. The open boxes represent a neomycin resistant gene (Neo) and a thymidine kinase gene (HSV-TK), respectively, and the open triangles represent loxp sequences. The arrows represent primers used in PCRs. Restriction enzymes are represented as follows: E, EcoRI; X, XbaI; RV, EcoRV; H, HindIII; N, NdeI.
Figure 2 shows the results of Southern blot analysis of cells obtained by performing homologous recombination in Example 1. The left shows the Southern blot of DNA prepared from offspring of MnSOD flox mice, and the right shows the result of genotype analysis by PCR. The symbols "wt" and "wild" mean a wild-type allele, and "lox" means a loxp allele. The terms "3'probe" and "Neo^{r} probe" represent the results obtained by using probes "3'probe" and "Neo^{r} probe" shown in Figure 3, respectively.
Figure 3 schematically shows the strategy for generation of liver-specific MnSOD-deficient mice (Example 2). The top line represents an allele after homologous recombination (loxp allele), and the bottom line represents recombination by Cre recombinase (del allele). Exon 3 is deleted from the bottom line. The solid boxes represent probes used in Southern blot analysis.
Figure 4 shows the results of Southern blot analysis of the liver-specific MnSOD-deficient mice. The terms "exon4 probe" and "Neo^{r} probe" represent the results obtained by using probes "exon4 probe" and "Neo^{r} probe" shown in Figure 3, respectively.
Figure 5 shows the result of Western blot analysis of the liver-specific MnSOD-deficient mice.
Figure 6 shows the results of measurement of SOD activities in the liver-specific MnSOD-deficient mice.
Figure 7 shows the results of HE staining of tissues from the liver-specific MnSOD-deficient mice. The terms "del/del" and "lox/lox" represent the liver-specific MnSOD-deficient mice and control mice, respectively.
Figure 8 shows the results of measurement of amounts of lipid peroxidation (MDA and 4-HAE) in the liver-specific MnSOD-deficient mice. The terms "del/del" and "lox/lox" represent the liver-specific MnSOD-deficient mice and control mice, respectively.

### BEST MODE FOR CARRYING OUT THE INVENTION

### (1) Knockin nonhuman animal of the present invention

The knockin nonhuman animal of the present invention has a DNA in which a region containing at least an exon of the MnSOD gene is interposed between two recombinase recognition sequences.

As the nonhuman animal, there may be mentioned, for example, mammals other than human (for example, mouse, rat, dog, cat, monkey, pig, cattle, sheep, goat, horse, or dolphin), birds (for example, chicken or quail), amphibians (for example, frog), or reptiles.

Base sequences of MnSOD genes from various nonhuman animals are known. For example, it is known that mouse MnSOD gene consists of five exons (exon 1 to exon 5) shown in Figure 1. The knockin nonhuman animal of the present invention may comprise one or more exons (preferably one exon) of the MnSOD gene, optionally with one or more introns adjacent to the exons. The exon which may be used in the present invention is not particularly limited, so long as deletion of the exon causes MnSOD deficiency. For example, exon 3 is preferable in a mouse.

In the present invention, a combination of a recombinase and a recognition sequence thereof is used. Recombinases carry out DNA recombination (site-specific recombination) by recognizing two specific short homologous regions (i.e., recombinase recognition sites) located in a DNA molecule. As the combination, there may be mentioned, for example, a Cre-loxp system or an FLP-FRT system. As the recombinase recognition site in the knockin nonhuman animal of the present invention, there may be mentioned, for example, a loxp sequence or an FRT sequence.

The knockin nonhuman animal of the present invention may further comprise a marker gene (preferably a drug resistance gene) between the two recombinase recognition sequences. The marker gene is not particularly limited, so long as the gene encodes a protein which can function as a drug resistance when a desired DNA is introduced into a knockin nonhuman animal. As the marker gene, there may be mentioned, for example, a neomycin (neo) resistance gene or a hypoxanthine phosphoribosyl transferase (HPRT) gene.

The knockin nonhuman animal of the present invention may be obtained by, but is by no means limited to, the following procedure. For example, a DNA in which a region containing at least one exon of the MnSOD gene is interposed between two recombinase recognition sequences (preferably a DNA in which a marker gene and a region containing at least one exon of the MnSOD gene are interposed between two recombinase recognition sequences) is constructed on an appropriate vector to prepare a targeting vector. The resulting targeting vector is used to carry out a homologous recombination in embryonic stem (ES) cells, and as a result, the knockin nonhuman animal of the present invention may be obtained. It may be confirmed whether or not the resulting nonhuman animal contains a desired DNA, for example, by Southern blot analysis.

The knockin nonhuman animal of the present invention includes a heterozygous nonhuman animal containing the DNA in which a region containing at least one exon of the MnSOD gene is interposed between two recombinase recognition sequences on either of homologous chromosomes, and a homozygous nonhuman animal containing the DNA on both of the homologous chromosomes.

### (2) Knockout nonhuman animal of the present invention

The knockout nonhuman animal of the present invention includes a knockout nonhuman animal in which MnSOD is specifically deficient in a specific tissue, and a knockout nonhuman animal in which the expression of MnSOD in a specific tissue is suppressed. The term "deficient" as used herein means that MnSOD does not substantially exist in a tissue. The term "suppressed" as used herein means that an amount of MnSOD expressed in a tissue is decreased in comparison with that in a wild-type, preferably 50% or less of that in a wild-type, more preferably 30% or less thereof, most preferably 20% or less thereof.

The knockout nonhuman animal of the present invention may be obtained, for example, by mating the knockin nonhuman animal of the present invention with a transgenic nonhuman animal expressing a recombinase in a specific tissue.

As the recombinase, there may be mentioned, for example, Cre recombinase or FLP recombinase.

For example, as a transgenic mouse expressing Cre recombinase in a specific tissue, there may be mentioned, for example,
Syn-Cre (expressing Cre recombinase in central nervous cells by a synapsin promoter);
Nes-Cre (expressing it in central and peripheral nerves by a nestin promoter);
Tek-Cre (expressing it in vascular endothelial cells by a Tie2 promoter);
MMTV-Cre (expressing it in mammary gland cells by a mouse mammary tumor virus LTR promoter)
PRM-Cre (expressing it in male gonad by a protamine promoter);
GZMB-Cre (expressing it in T cells by a granzyme promoter);
Alb-Cre (expressing it in the liver by an albumin promoter);
Ins2-Cre (expressing it in the pancreas by an insulin 2 promoter);
CoII I-Cre (expressing it in cartilage cells by a type II collagen promoter);
[The above mice are available from The Jackson Laboratory, 600 Main Street, Bar Harbor, ME 04609, USA]
PB-Cre (expressing it in the prostate by a probasin promoter);
MyHC-Cre (expressing it in cardiac muscles by a myosin heavy chain promoter);
[The above mice are available from Baylor College of Medicine, One Baylor Plaza, Houston, TX 77030, USA]
TH-Cre (expressing it in dopaminergic neurons by a tyrosine hydroxylase promoter; available from Department of Molecular Genetics, Institute of Biomedical Sciences, Fukushima Medical University School of Medicine); or
K14-Cre (expressing it in epidermis by a keratin 14 promoter; available from Strasbourg University, I.G.B.M.C. CNRS-INSERM-UPL, BP 163, 67404 ILLKIRCH CEDEX C.U. STRASBOURG, France).

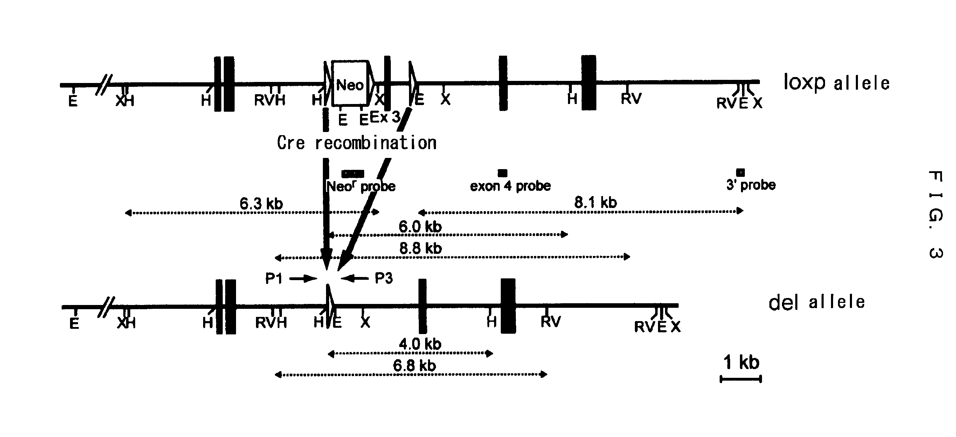

When the knockin nonhuman animal of the present invention is mated with a transgenic nonhuman animal expressing a recombinase in a specific tissue, the region interposed between two recombinase recognition sequences (i.e., the region containing one or more exons of the MnSOD gene) is excised by the recombinase in the specific tissue, as shown in Figure 3, but the excision does not occur in tissues where the recombinase is not expressed. Therefore, in the knockout nonhuman animal of the present invention obtained by the mating, the expression of MnSOD is specifically suppressed or MnSOD is specifically deficient only in the tissue where the recombinase is expressed, and the expression of MnSOD in the other tissues does not change from that of the wild-type.

In this connection, when a wild-type nonhuman animal with respect to the MnSOD gene is used as the transgenic nonhuman animal expressing a recombinase, the knockout nonhuman animal of the present invention in which the expression of MnSOD in the specific tissue is suppressed (i.e., heterozygote) may be obtained. In contrast, when a transgenic nonhuman animal deficient in the MsSOD gene is used as the transgenic nonhuman animal expressing a recombinase, the knockout nonhuman animal of the present invention in which MnSOD is specifically deficient in the specific tissue may be obtained.

Further, the knockout nonhuman animal of the present invention obtained by mating the knockin nonhuman animal of the present invention and the transgenic nonhuman animal expressing a recombinase in a specific tissue, is mated with the same or a different knockin nonhuman animal of the present invention to obtain the knockout nonhuman animal of the present invention in which MnSOD is specifically deficient in the specific tissue (i.e., homozygote). Alternatively, the knockout nonhuman animal of the present invention obtained by mating the knockin nonhuman animal of the present invention and the transgenic nonhuman animal expressing a recombinase in a specific tissue, is mated with the knockout nonhuman animal of the present invention obtained in a similar fashion to obtain the knockout nonhuman animal of the present invention in which MnSOD is specifically deficient in the specific tissue (i.e., homozygote).

It may be confirmed whether or not the nonhuman animal obtained by the mating is deficient in one or more exons of the MnSOD gene, for example, by Southern blot analysis. Further, it may be confirmed whether or not MnSOD is deficient in the specific tissue, for example, by Western blot analysis.

### EXAMPLES

The present invention now will be further illustrated by, but is by no means limited to, the following Examples.

### EXAMPLE 1

In the present example, knockin mice in which exon 3 of the MnSOD gene was interposed between two loxp sequences were generated, as shown in Figure 1.

A 129/Sv mouse genome library (λFIXII; Stratagene) and mouse MnSOD cDNA as a probe were used to clone an approximately 14 kb genomic gene containing five exons of MnSOD into a pBSKII vector (Stratagene).

The isolated MnSOD genomic gene was used as a template to amplify a 5' flanking 1.3 kb genome fragment by the PCR method using a primer set of an NotI anchored sense primer (SEQ ID NO: 1) and an SalI anchored antisense primer (SEQ ID NO: 2), and the resulting fragment was cloned into a pMC1DT-A(B) plasmid (Oriental Koubo).

The isolated MnSOD genomic gene was used as a template to amplify a 3' flanking approximately 6 kb genome fragment by the PCR method using a primer set of an XhoI anchored sense primer (SEQ ID NO: 3) and an SalI anchored antisense primer (SEQ ID NO: 4). The resulting fragment was cloned, at the SalI and XhoI sites, into the previously obtained pMC1DT-A(B) plasmid containing the 5' flanking genomic gene.

A plasmid pMCneo-loxp vector comprising the neomycin resistance gene flanked by loxp sequences [provided by Dr. Yagi, Osaka University; Nucleic Acids Res., 26 (1998) 679-680] was used to insert the neomycin resistance gene flanked by loxp sequences into a pBSKII vector at the XhoI/SalI sites and prepare a Neo^{r}/BSK vector. The third loxp sequence was inserted into the Neo^{r}/BSK vector to prepare a Neo^{r}-loxp/BSK vector.

The isolated MnSOD genomic gene was used as a template to amplify a genome fragment containing exon 3 and the adjacent regions by the PCR method using a primer set of an SalI anchored sense primer (SEQ ID NO: 5) and an SalI anchored antisense primer (SEQ ID NO: 6), and the resulting fragment was cloned into the Neo^{r}-loxp/BSK vector at the SalI site. The fragment containing exon 3 and the neomycin resistance gene was isolated using restriction enzymes KpnI and SmaI, blunted, and cloned into the previously obtained pMC1DT-A(B) vector containing the 5' and 3' flanking genomic genes. The targeting construct was excised from the resulting vector using restriction enzymes NotI and BamHI, and cloned into a PIC19R/MCI-TK vector [provided from Dr. Capecchi, University of Utah; Nature, 336 (1988) 348-352 or Science, 301 (2003) 363-367] to prepare a knockin construct (Figure 1, targeting vector).

The resulting targeting construct was digested with restriction enzyme NotI, and the linearized targeting construct was incorporated into ES cells by the electropolation method.

Homologous recombinant cells cultured in a medium containing G418 were confirmed by the Southern blot method as described below.

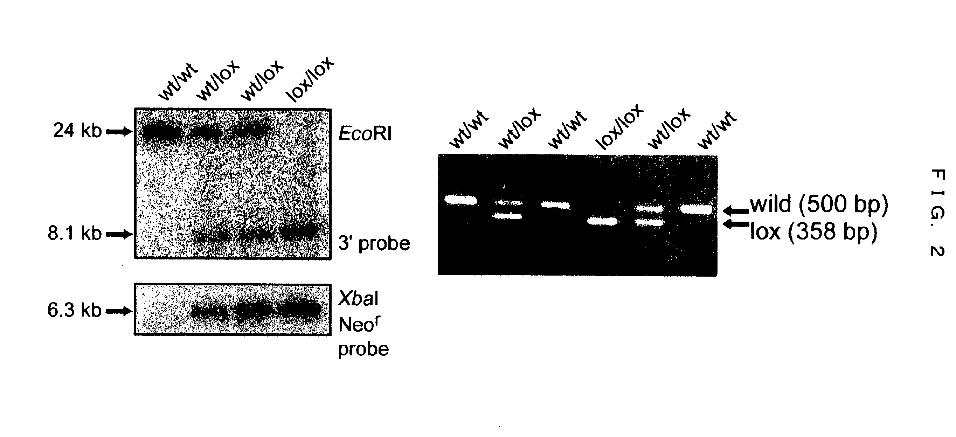

Each DNA prepared from postnatal offspring was digested with restriction enzyme EcoRI. Using 3'probe, 24 kb fragment and 8.1 kb fragment were detected in the wild-type mouse (wt/wt) and the homozygous MnSOD flox mouse (lox/lox), respectively (Figure 2, upperleft). When the DNAs were digested with restriction enzyme XbaI and the Neo^{r} probe was used, 6.3 kb fragment was detected in the homozygous mouse (lox/lox) and the heterozygous mouse (wt/lox or lox/wt), but no fragment was detected in the wild-type mouse (wt/wt) (Figure 2, lowerleft).

Further, chimeric mice were prepared to confirm the deletion in the germline by the Southern blot method. The PCRs were carried out using DNAs extracted from offspring tails and primers P1 (SEQ ID NO: 7), P2 (SEQ ID NO: 8), and P4 (SEQ ID NO: 9) shown in Figure 1. As shown in Figure 2 (right), 500 bp fragment and 358 bp fragment were detected in the wild-type allele (wt/wt) and mutated alleles (wt/lox, lox/wt, or lox/lox), respectively.

### EXAMPLE 2

In the present example, model mice in which MnSOD was specifically deficient in liver cells were generated and analyzed.

Rat albumin promoter (Alb)-Cre transgenic mice (THE JACKSON LABORATORY, 600 MAIN STREET, BAR HARBOR, ME 04609, USA) expressing Cre recombinase in hepatocytes were mated with the MnSOD flox mice generated in Example 1, as shown in Figure 3.

As a result, liver-specific MnSOD-deficient mice were not lethal, and outwardly, were not different from the wild-type.

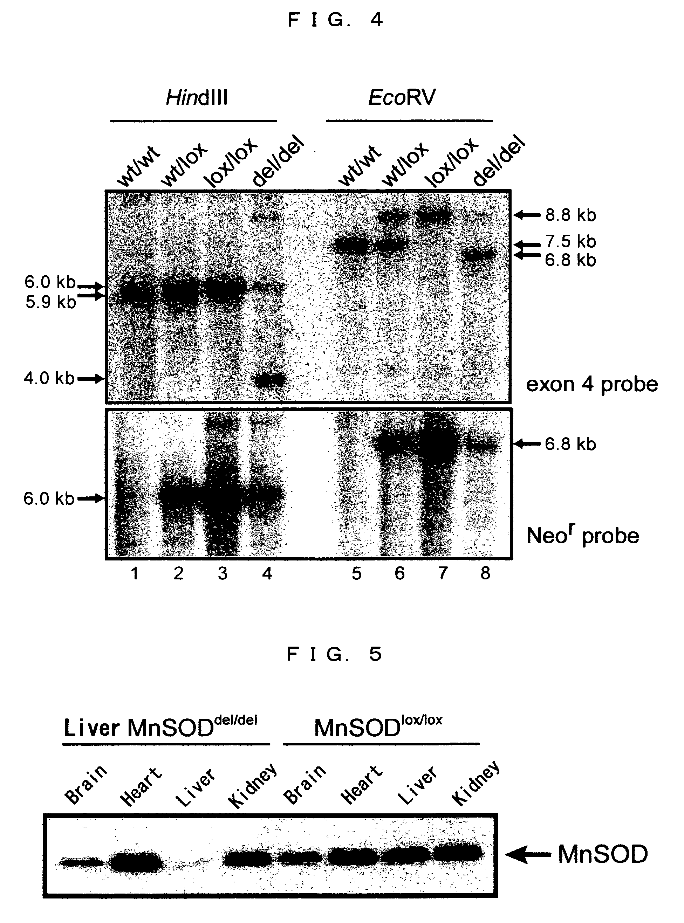

The DNAs obtained from livers were used to confirm the efficiency of the recombination by the Southern blot method (Figure 4).

When restriction enzyme HindIII and the exon4 probe were used, 6.0 kb DNA fragment and 4.0 kb DNA fragment were detected in the MnSOD flox allele (lox/lox) and MnSOD-deficient allele (del/del), respectively.

When restriction enzyme EcoRV and the exon4 probe were used, 8.8 kb DNA fragment and 6.8 kb DNA fragment were detected in the MnSOD flox allele and MnSOD-deficient allele, respectively. Each DNA contained a small amount of DNA fragment in which recombination by Cre did not occur. When DNAs prepared from the homozygous or heterozygous MnSOD flox allele mice and the Neo^{r} probe were used, 6.1 kb and 6.8 kb fragments were detected in the DNAs digested with restriction enzymes HindIII and EcoRV, respectively (no fragment was detected in the wild-type). In the MnSOD-deficient allele, a small amount of DNA fragment was detected, as it was in the case of the exon4 probe.

From the above results, it was confirmed that exon3 of the MnSOD gene was almost deleted by the genetic recombination, as expected.

In this connection, each sample extracted from the brain, heart, liver, or kidney of the liver-specific MnSOD-deficient mice was analyzed by the Western blot method. As a result, it was confirmed that MnSOD was deficient in only the liver, and that no difference was observed in the other organs (Figure 5).

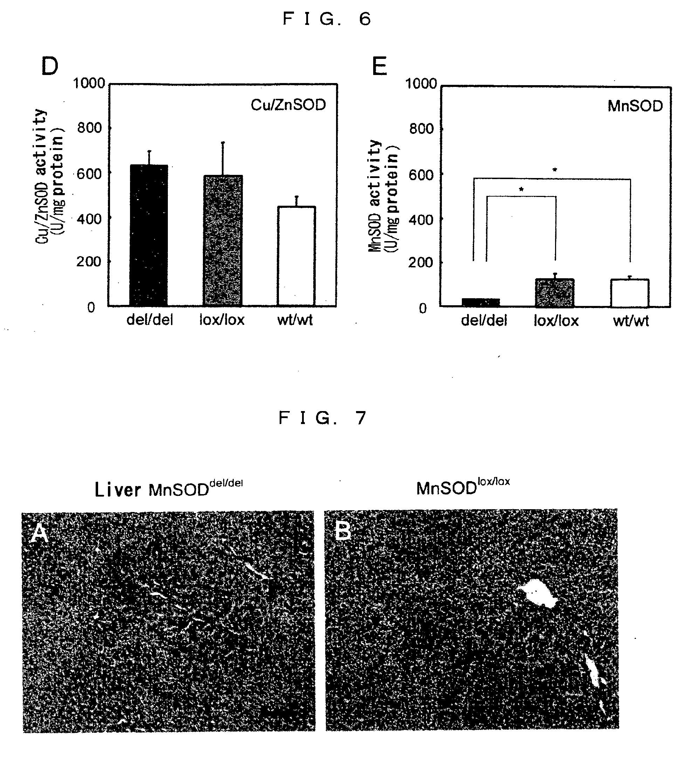

Next, the SOD activity in each liver of the liver-specific MnSOD-deficient mice or control mice was measured (Figure 6). No significant difference in the Cu/ZnSOD activity was observed (Figure 6, left), but the MnSOD activity in the liver-specific MnSOD-deficient mice was decreased by 70% in comparison with the control (Figure 6, right).

### EXAMPLE 3

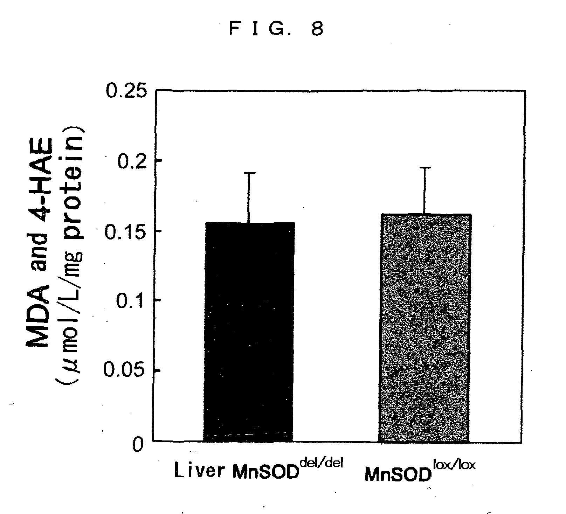

It was reported that fats and glycogen were accumulated in the liver of the MnSOD-deficient mice caused by the MnSOD deficiency. Therefore, in the present example, the liver-specific MnSOD-deficient mice generated in Example 2 were used to carry out the hematoxylin-eosin (HE) staining, periodic acid-Schiff (PAS) staining, and Oil Red O staining. As a result, no significant difference was observed in the accumulation of glycogen (Figure 7).

Further, on the basis of the expectation that organs in the deficient mice are exposed to reactive oxygen species more than in the wild-type, amounts of lipoperoxides [malondialdehyde (MDA) and 4-hydroxy-alkenals (4-HAE)] were measured. No significant difference was observed in comparison with the control (Figure 8).

From the above results, it was suggested that the changes observed in the liver of the conventional MnSOD-deficient mouse was caused by the deficiency of MnSOD in the whole body, but not by the deficiency of MnSOD in the liver.

### INDUSTRIAL APPLICABILITY

The present inventors succeeded in generating model mice that enabled the observation of the destruction of protection against reactive oxygen species in each organ separately by the gene manipulation and the effects of reactive oxygen species against the living body. The tissue-specific MnSOD-deficient mouse of the present invention is different from the conventional MnSOD-deficient mice, and is tissue-specifically deficient in the MnSOD activity, and thus, it is possible to avoid the lethal effects occurring in the neonatal period. According to the model mouse of the present invention, it is possible to observe senescence and aging in each organ separately. Such an observation was not possible using the conventional model mice. Further, according to the model mouse of the present invention, the sensitivity of each organ to reactive oxygen species can be analyzed, and the sensitivity to senescence can be evaluated in each organ separately.

The model mouse of the present invention is essential to the analysis of diseases in which morbidity is increased with senescence and specific changes occur in an organ (for example, Parkinson disease or arteriosclerosis). Further, the model mouse is essential to the development of tissue-specific agents for treating such a disease, for example, an agent for preventing senescence in the brain. Furthermore, the present invention provides an assay system useful for checking the effects of developed agents.

### FREE TEXT IN SEQUENCE LISTING

Features of "Artificial Sequence" are described in the numeric identifier <223> in the Sequence Listing. More particularly, each of the base sequences of SEQ ID NOS: 1 to 6 is an artificially synthesized primer sequence.

Although the present invention has been described with reference to specific embodiments, various changes and modifications obvious to those skilled in the art are possible without departing from the scope of the appended claims.

## Claims

1. A knockin nonhuman animal having a DNA wherein a region containing at least an exon of a manganese superoxide dismutase gene is interposed between two recombinase recognition sequences.

2. The knockin nonhuman animal according to claim 1, wherein the exon is exon 3.

3. The knockin nonhuman animal according to claim 1 or 2,
wherein a marker gene is interposed between the two recombinase recognition sequences.

4. The knockin nonhuman animal according to claim 3, wherein the marker gene is a neomycin resistance gene.

5. The knockin nonhuman animal according to any one of claims 1 to 4, wherein the recombinase recognition sequence is a loxp sequence.

6. The knockin nonhuman animal according to any one of claims 1 to 5, wherein the nonhuman animal is a mouse.

7. A knockout nonhuman animal obtainable by mating a transgenic nonhuman animal expressing a recombinase in a specific tissue with the knockin nonhuman animal according to any one of claims 1 to 6, wherein an expression of manganese superoxide dismutase in the specific tissue is suppressed or deficient.

8. A knockout nonhuman animal obtainable by mating a first knockout nonhuman animal according to claim 7, with the knockin nonhuman animal according to any one of claims 1 to 6 or a second knockout nonhuman animal according to claim 7, wherein manganese superoxide dismutase is specifically deficient in the specific tissue.

9. The knockout nonhuman animal according to claim 7 or 8, wherein the recombinase is Cre recombinase.

10. A DNA wherein a region containing at least an exon of a manganese superoxide dismutase gene is interposed between two recombinase recognition sequences.
